# EUROPÄISCHE PATENTSCHRIFT

(11) **EP 2 389 242 B1**
(45) Veröffentlichungstag und Bekanntmachung des Hinweises auf die Patenterteilung: **21.10.2015**
(21) Anmeldenummer: 10706472.7
(22) Anmeldetag: 20.01.2010
(51) Int. Cl.: A61M 1/36, B01D 15/00, B01D 15/08, B01J 20/28, B01J 20/32

(54) **SORPTIONSMITTEL FÜR ENDOTOXINE**
SORBENT FOR ENDOTOXINS
SORBENT POUR ENDOTOXINS

(30) Priorität: 22.01.2009 AT 1112009
(43) Veröffentlichungstag der Anmeldung: 30.11.2011
(73) Patentinhaber: Fresenius Medical Care Deutschland GmbH, 61352 Bad Homburg (DE)
(72) Erfinder: FALKENHAGEN, Dieter, A-3500 Krems (AT); WEBER, Viktoria, A-3500 Krems/Donau (AT)
(74) Vertreter: Patentanwaltskanzlei Matschnig & Forsthuber OG
(86) Internationale Anmeldenummer: PCT/AT2010/000017
(87) Internationale Veröffentlichungsnummer: WO 2010/083545

(56) Entgegenhaltungen:
- WO-A1-2007/142611
- US-A- 5 510 242
- BLAIS B W ET AL: "Use of polymyxin-coated polyester cloth in the enzyme immunoassay of Salmonella lipopolysaccharide antigens" INTERNATIONAL JOURNAL OF FOOD MICROBIOLOGY, ELSEVIER SCIENCE PUBLISHERS, AMSTERDAM, NL LNKD- DOI:10.1016/0168-1605(90)90012-T, Bd. 11, Nr. 3-4, 1. Dezember 1990 (1990-12-01), Seiten 195-204, XP023697820 ISSN: 0168-1605 [gefunden am 1990-12-01]
- BRANDL M ET AL: "Particle Transfer and Detection in a Microspheres Based Detoxification System" BIOCOMPUTATION, BIOINFORMATICS, AND BIOMEDICAL TECHNOLOGIES, 2008. BIOTECHNO '08. INTERNATIONAL CONFERENCE ON, IEEE, PISCATAWAY, NJ, USA, 29. Juni 2008 (2008-06-29), Seiten 120-124, XP031284095 ISBN: 978-0-7695-3191-5

## Beschreibung

Die Erfindung betrifft ein Sorptionsmittel zum Entfernen von Endotoxinen aus einer biologischen Flüssigkeit, wobei das Sorptionsmittel einen wasserunlöslichen, porösen Träger und Polymyxin, welches am Träger immobilisiert ist, aufweist, wobei der Träger eine nichtionische, hydrophobe Oberfläche aufweist und Polymyxin über hydrophobe Interaktion an der Oberfläche des Trägers immobilisiert ist.

Endotoxine sind Lipopolysaccharide (LPS) in der Zellwand gramnegativer Bakterien und werden durch Zelllyse freigesetzt. In der Tat sind Lipopolysaccharide der häufigste Lipidbestandteil der äußeren Zellmembran gramnegativer Bakterien. Endotoxine sind pyrogene Substanzen, d.h. das betroffene Individuum reagiert mit einer starken Entzündungsreaktion und Fieber, wenn Endotoxine, beispielsweise im Zuge einer mikrobiellen Vergiftung, in den Körper gelangen und systemische Wirkung zeigen. Die Anwesenheit von Endotoxinen im Blutkreislauf führt zu einer unkontrollierten Aktivierung der Immunzellen und einem Ungleichgewicht des Gerinnungssystems. In Abhängigkeit ihrer Konzentration können sie zu einer Sepsis führen, welche unter anderem durch hohes Fieber, niedrigen Blutdruck und, in schweren Fällen, durch Multiorganversagen gekennzeichnet ist. Eine Sepsis ist eine sehr ernstzunehmende Erkrankung; die Letalität von Individuen mit schwerer Sepsis oder septischem Schock ist je nach Schweregrad der Erkrankung ca. 30-60%. Auch Patienten mit beeinträchtigter Immunabwehr, wie z.B. Leberpatienten oder Patienten in Chemotherapie, neigen zu bakteriellen Infektionen und zeigen dadurch Symptome einer Endotoxinvergiftung.

Die Struktur eines Lipopolysaccharid-Moleküls ist dreiteilig: Ein Lipid A bildet den Bereich des Moleküls, welcher der Bakterienzelle zugewandt ist; durch das Lipid A wird das Molekül in der äußeren Membran des gramnegativen Bakteriums verankert. Das LPS-Molekül weist ferner eine mittlere, hoch konservierte Kernregion auf, welche an das Lipid A gebunden ist. Der dritte und äußerste Bereich wird durch ein O-spezifisches Polysaccharid (O-Antigen) gebildet, dessen Struktur innerhalb der verschiedenen gramnegativen Bakterien stark variieren kann. Die toxische Wirkung ist auf das Lipid A zurückzuführen, welches erst bei der Zelllyse freigesetzt wird.

Endotoxine können mittels eines geeigneten Sorptionsmittels aus einer mit Endotoxinen verunreinigten biologischen Flüssigkeit wie Blut oder Plasma entfernt werden. Die Behandlung von Patienten mit Endotoxinvergiftungen bzw. Sepsis findet insbesondere im Rahmen einer extrakorporalen Blutreinigung (Apherese) statt.

Aphereseverfahren sind extrakorporal durchgeführte Verfahren, bei welchen pathophysiologisch relevante Blut- und Plasmakomponenten, beispielsweise Biomoleküle wie (Glyko)Proteine, Peptide, Lipide, Lipoproteine und Lipopolysaccharide, aber auch Blutzellen und Blutplasma entfernt werden. Aphereseverfahren können einerseits zu diagnostischen und therapeutischen Zwecken eingesetzt werden, andererseits stellen sie auch eine sehr effektive Möglichkeit dar, bestimmte Blutbestandteile von gesunden Individuen in ausreichender Menge und in ausreichend hoher Reinheit zu gewinnen. Große Bedeutung wird der therapeutischen Apherese zugemessen, da diese bei bestimmten Indikationen oft eine sehr wirkungsvolle und gleichzeitig nebenwirkungsarme Alternative zur medikamentösen Behandlung ist. So kann bei Plasmaphereseverfahren das Plasma entweder vollständig abgetrennt und durch eine Substitutionslösung ersetzt werden, oder es werden nur bestimmte Bestandteile wie LDL, Endotoxine oder Immunglobuline mittels eines Sorptionsmittels daraus entfernt und das Plasma anschließend in den Spender/Patienten wieder zurückgeführt.

Zum Entfernen von Endotoxinen aus einer mit Endotoxinen verunreinigten biologischen Flüssigkeit (zumeist Blut oder Blutplasma) wird diese mit einem Sorptionsmittel, welches sich üblicherweise in einer Sorptionseinrichtung befindet, in engen Kontakt gebracht. Die Endotoxine werden an der Oberfläche des Sorptionsmittels gebunden und aus der biologischen Flüssigkeit entfernt. Die von Endotoxinen befreite biologische Flüssigkeit wird dem Patienten wieder zugeführt. Die Sorptionseinrichtung ist entweder in einem extrakorporalen Blutkreislauf blutseitig oder in einem Plasmakreislauf einer extrakorporalen Blutreinigungsvorrichtung filtratseitig angeordnet. Die Endotoxinbindungskapazität und -geschwindigkeit ist dabei von der Beschaffenheit des Sorptionsmittels abhängig.

Die Geschwindigkeit der Endotoxinbindung durch das Sorptionsmittel ist entscheidend für das Überleben des Patienten. Die Zeit, die bleibt, um die Endotoxine aus dem Blut eines Patienten zu entfernen, ist sehr kurz (< 12 h) und kann bei schwerer Sepsis lediglich einige Stunden betragen.

Es hat sich gezeigt, dass Anionenaustauscherharze (z.B. DEAE oder PEI-Gruppen an Cellulose gebunden) sehr gut für die Endotoxin-Bindung geeignet sind. Nachteilig ist jedoch die unerwünschte Bindung wichtiger Faktoren des intrakorporalen Gerinnungssystems wie Protein C und Protein S und die damit verbundene Gerinnungsproblematik.

Diese Gerinnungsproblematik kann durch die Verwendung eines spezifischen Sorptionsmittels, welches immobilisierte Antikörper gegen Endotoxine aufweist, umgangen werden. Diese Möglichkeit ist jedoch aus ökonomischen Gründen nur limitiert anwendbar.

Als sehr nützliche Alternative sind Sorptionsmittel der eingangs genannten Art, bei welchen Polymyxin-Moleküle auf einem wasserunlöslichen, porösen Träger immobilisiert sind, bekannt geworden.

Polymyxine sind antibiotische Substanzen zur Behandlung von Infektionen mit gram-negativen Bakterien. Polymyxine greifen in die Zellwandstruktur ein, indem sie die Permeabilität der Zellmembran erhöhen, aufgrund dessen es zur Zelllyse kommt. Polymyxine binden nicht nur Phospholipide, sondern auch Lipopolysaccharide (Endotoxine) mit hoher Affinität. Aufgrund der neurotoxischen und nephrotoxischen Wirkung der Polymyxine haben nur Polymyxin B und Polymyxin E (Colistin) gewisse therapeutische Bedeutung erlangt. Polymyxin B bzw. Polymyxin E sind daher lediglich für orale oder topische Therapieformen anwendbar. Für eine parenterale, systemische Behandlung von Endotoxinvergiftungen bzw. Sepsis sind sie aufgrund ihrer toxischen Wirkung nicht geeignet.

Es hat sich jedoch als günstig erwiesen, Polymyxin, insbesondere Polymyxin B, auf einem wasserunlöslichen Träger kovalent zu binden und den Polymyxin B-beschichteten Träger als Sorptionsmittel zum Entfernen von Endotoxinen aus verunreinigten biologischen Flüssigkeiten einzusetzen.

In der EP 0110 409 A sind Polymyxin B-immobilisierte Träger aus porösem Glas (FPG 2000) sowie Polymyxin B-immobilisierte Polysaccharid-Träger auf Zellulose-Basis (Cellulofine A-3) offenbart. Ebenso bekannt sind Mikropartikel aus Zellulose oder derivatisierter Zellulose, an welche Polymyxin B kovalent gebunden ist. [Weber V., Loth F., Linsberger L, Falkenhagen D.: Int. J. Artif. Organs 25(7), 679] Mit Polymyxin-beschichteten Zellulose-Trägern konnte eine hohe Endotoxin-Adsorption erreicht werden, sie haben jedoch den Nachteil, dass Polymyxin an diese Träger kovalent gebunden und die Zellulose folglich vor der Bindung des Polymyxins chemisch aktiviert werden muss.

Die EP 0 129 786 A2 beschreibt ein Endotoxin-Entgiftungsmaterial mit einem faserartigen Träger, an welchem Polymyxin kovalent immobilisiert ist. Der faserartige Träger ist mit funktionellen Gruppen ausgestattet ist, um Polymyxin kovalent an die Oberfläche des Trägers zu binden. Das Endotoxin-Entgiftungsmaterial aus der EP 0129 786 ist als Füllmaterial für ein Adsorptionsmodul (Handelsname: Toraymyxin) [Shoji H. 2003. Extrakorporeal endotoxin removal for the treatment of sepsis: endotoxin adsorption cartridge (Toraymyxin)] auf dem Markt und im Augenblick das einzige Sorptionsmittel, welches für die klinische Behandlung der Sepsis im Rahmen einer extrakorporalen Blutreinigung zugelassen ist. Ein kritischer Review über die Effektivität von faserartigen Trägern mit immobilisiertem Polymyxin B, in welchem die Qualität der Behandlung als suboptimal dargestellt wird, ist erst kürzlich publiziert worden [Cruz DN et al. 2007; Effektiveness of polymyxin B-immobilized fiber column in sepsis: a systematic review. Crit. Care 11(3):137].

Nachteilig an den bekannten Sorptionsmitteln, die auf einer Bindung der Endotoxine durch Polymyxin beruhen, ist die geringe Endotoxinbindungskapazität- und geschwindigkeit. Da Polymyxin über NH₂-Gruppen am Polymer gebunden ist, ist der Zugang für Endotoxine beeinträchtigt. Weitere Nachteile ergeben sich aus dem aufwändigen und komplizierten Herstellungsverfahren und den damit verbundenen höheren Herstellungskosten.

WO 2007/142611 A1, US 5,510,242 und Blais et al. [Blais BW, Yamazaki H, 1990. International Journal of Food Microbiology 11:195-204] offenbaren einen porösen Träger mit einer neutralen, hydrophoben Oberfläche, an welcher Polymyxin B über hydrophobe Interaktion gebunden ist.

Brandl et al. [Brandl M et al. 2008. International Conference on Biocomputation, Bioinformatics, and Biomedical Technologies. IEEE, Piscataway, USA. S. 120-124] offenbaren die Verwendung Mikropartikel-förmiger Sorptionsmittel in einem Microspheres-based Detoxification System (MDS).

Obwohl die Letalität von Patienten mit Endotoxinvergiftungen, insbesondere Sepsis, durch die klinische Anwendung des oben genannten Toraymyxin-Adsorptionsmoduls gesenkt werden konnte, ist die Letalität von Patienten mit schwerer Sepsis und septischen Schock trotz maximaler Therapie immer noch sehr hoch. Aus diesem Grund sowie aufgrund der hohen und ansteigenden Inzidenz von septischen Zuständen besteht ein hoher Bedarf an einem Sorptionsmittel mit verbesserter Sorptionsleistung für Endotoxine.

Es ist eine Aufgabe der Erfindung, ein Sorptionsmittel bereitzustellen, mit welchem Endotoxine in hohem Ausmaß und mit hoher Geschwindigkeit aus einer biologischen Flüssigkeit entfernt werden können.

Die Aufgabe wird durch ein Sorptionsmittel der eingangs genannten Art gelöst, bei welchem erfindungsgemäß der Träger eine mittlere Porengröße von 80 - 100 nm aufweist, wobei sich die mittlere Porengröße auf den mittleren Durchmesser der Poren bezieht.

Dank des erfindungsgemäßen Sorptionsmittels konnte eine deutliche Verbesserung der Endotoxinsorptionskapazität und Endotoxinsorptionsgeschwindigkeit erreicht werden. Die Erfinder haben in unerwartetem Maße festgestellt, dass - im Vergleich zu den bekannten Sorptionsmitteln - durch das erfindungsgemäße Sorptionsmittel bereits nach einer kurzen Einwirkzeit eine große Endotoxinmenge aus einer Endotoxin-verunreinigten biologischen Flüssigkeit gebunden werden kann. Dies hat große therapeutische Vorteile, insbesondere für Patienten mit Sepsis, da ein großes Volumen an biologischer Flüssigkeit in kurzer Zeit von Endotoxinen befreit werden kann. Durch das erfindungsgemäße Sorptionsmittel können die Überlebenschancen von Patienten mit schwerer Sepsis verbessert werden. Wie bereits erwähnt ist die Geschwindigkeit der Endotoxinbindung durch das Sorptionsmittel entscheidend für das Überleben des Patienten. Auch die Behandlungsdauer im Rahmen einer extrakorporalen Blutreinigung kann dank des erfindungsgemäßen Sorptionsmittels verkürzt werden, wodurch zeitliche, finanzielle und humane Ressourcen eingespart werden können. Ein weiterer Vorteil der Erfindung gegenüber den bekannten Sorptionsmitteln ist dessen besonders einfache Herstellung, da Polymyxin (mittels des hydrophoben Abschnitts des Polymyxin-Moleküls) über hydrophobe Interaktion an der neutralen, hydrophoben Oberfläche des Trägers immobilisiert wird. Die Steigerung der Endotoxinbindungseffektivität des erfindungsgemäßen Sorptionsmittels lässt sich dadurch erklären, dass die mittels hydrophober Interaktion vermittelte Bindung die NH₂-Gruppen des Polymyxins freilässt und diese im Wesentlichen in ihrer Gesamtheit für die Endotoxinbindung zur Verfügung stehen. Es sind keine komplexen chemischen Schritte zum Immobilisieren des Polymyxins an den Träger notwendig. Die Herstellung des erfindungsgemäßen Sorptionsmittels kann daher ökonomisch und reproduzierbar durchgeführt werden.

Die hydrophobe Interaktion, auch hydrophobe Wechselwirkung genannt, besitzt große biochemische Bedeutung und beruht auf dem Phänomen, dass hydrophobe Moleküle in einer polaren Umgebung zur Assoziation neigen. Die hydrophobe Interaktion ist daher keine Kraft per se, sondern wird durch eine polare Umgebung erzwungen. Bei der vorliegenden Erfindung findet die hydrophobe Interaktion zwischen dem hydrophoben Abschnitt des Polymyxin-Moleküls und den neutralen, hydrophoben inneren und äußeren Oberflächen des porösen Trägers statt.

Unter dem Begriff "Sorptionsmittel" ist im Rahmen dieser Offenbarung ein Mittel zum Durchführen einer Sorption, vorzugsweise einer Adsorption zu verstehen, d.h. Moleküle, die sich in einer biologischen Flüssigkeit befinden, werden durch die Oberflächenkräfte des Sorptionsmittels festgehalten. In der Beschreibung werden daher anstelle des Begriffs "Sorptionsmittel" auch die Begriffe "Adsorbtionsmittel" bzw. "Adsorbens" bzw. "Adsorber" verwendet. Erfindungsgemäß ist das Sorptionsmittel zur Adsorption von Endotoxinen aus einer mit Endotoxinen verunreinigten, biologischen Flüssigkeit vorgesehen. Anwendung findet das erfindungsgemäße Sorptionsmittel vor allem bei der extrakorporalen Blutreinigung, insbesondere bei Patienten mit septischen Zuständen.

Der im Rahmen der Erfindung verwendete Ausdruck "biologische Flüssigkeit" kann sich auf zellfreie Flüssigkeiten, insbesondere Blutplasma, oder auf Zellen enthaltende Flüssigkeiten, insbesondere Blut, beziehen. Da es im Zuge einer extrakorporalen Blutreinigung auch notwendig ist, andere Flüssigkeiten wie beispielsweise Gerinnungshemmer enthaltende Lösungen (Heparin-Lösung, Citrat-Lösung) oder Substitutionslösungen (Elektrolyte, Flüssigkeiten zum Ausgleich des Flüssigkeitsverlust) in den extrakorporalen Blutkreislauf bzw. in einen Blutplasmakreislauf einzubringen, ist unter einer biologischen Flüssigkeit auch verdünntes Blut bzw. verdünntes Blutplasma zu verstehen. Die Erfindung ist hauptsächlich für den humanmedizinischen Bereich angedacht und bezieht sich daher auf menschliche biologische Flüssigkeiten. Dies schließt jedoch die Anwendung der Erfindung im veterinärmedizinischen Bereich nicht aus.

Polymyxine sind bekannte chemische Verbndungen, welche ursprünglich aus dem Bakterium *Bacillus polymyxa* stammen. Insbesondere sind Polymyxin B und Polymyxin E (Colistin) hervorzuheben.

Der Begriff "wasserunlöslicher, poröser Träger, der eine nicht-ionische, hydrophobe Oberfläche aufweist" wie im unabhängigen Anspruch 1 verwendet bezieht sich im Rahmen dieser Offenbarung auf einen porösen, wasserunlöslichen Feststoff, der äußere und innere Oberflächen aufweist. Die äußeren und inneren Oberflächen sind neutral und hydrophob. Unter dem Begriff "neutral" ist nicht-ionisch zu verstehen. Die Erfindung ist vor allem auf partikelförmige Träger ausgerichtet.

Für die Praxis ist es besonders zweckmäßig, wenn der Träger ein hydrophobes Polymer ist. Dadurch kann eine gute Reproduzierbarkeit des Trägermaterials gewährleistet werden, insbesondere hinsichtlich der Porosität und der Partikelgröße. Die Porosität und die Partikelgröße lassen sich darüber hinaus sehr gut variieren. Beim hydrophoben Polymer kann es sich sowohl um ein Homopolymer als auch um ein Heteropolymer handeln. Für die praktische Durchführung haben sich vernetzte Polystyrol-Polymere als besonders günstig erwiesen. Bei der extrakorporalen Blutreinigung bestehen hohe Anforderungen an die Sterilität der Vorrichtungsbestandteile, welche mit den Körperflüssigkeiten des Patienten in Kontakt kommen. Dies gilt auch für Sorptionsmittel. Vernetzte Polystyrol-Polymere zeichnen sich durch eine hohe Stabilität gegenüber Hitze und Chemikalien aus und sind in der klinischen Praxis bereits etabliert.

Die Stärke der hydrophoben Interaktion zwischen Polymyxin und Träger ist einerseits durch die Hydrophobizität des neutralen, hydrophoben Trägers und andererseits durch die Ionenstärke des Mediums bestimmt. Wie oben bereits erwähnt wurde, hat Polymyxin neurotoxische und nephrotoxische Wirkung. Es ist daher eine möglichst feste Bindung des Polymyxins an die äußeren und inneren Oberflächen des Trägers erwünscht. Bei einer besonders bevorzugten Variante ist das vernetzte Polystyrol-Polymer ein Polystyrol-Divinylbenzol-Copolymer. Die Oberfläche eines Polystyrol-Divinylbenzol-Copolymers weist eine hohe Hydrophobizität auf, wodurch eine sehr starke Bindung des Polymyxins an die Trägeroberfläche erreicht wird. Die Erfinder haben festgestellt, dass nach dem Immobilisieren des Polymyxins über hydrophobe Interaktion kein Polymyxin in die biologische Flüssigkeit freigesetzt wird. Es ist jedoch auch möglich, andere neutrale, hydrophobe Polymere hoher Hydrophobizität zu verwenden, welche einem einschlägigen Fachmann gut bekannt sind. Die Erfinder konnten feststellen, dass beim Autoklavieren des erfindungsgemäßen Sorptionsmittels keine Desorption des Polymyxins und somit auch keine Einbuße der Endotoxinbindung messbar ist. Dies ist für die Patientensicherheit von großem Vorteil.

Weiters wurde gefunden, dass auch die Porengröße des porösen Trägers hinsichtlich der Endotoxinadsorption von Bedeutung ist. Es ist daher günstig, auch aus Gründen der Reproduzierbarkeit, wenn der poröse Träger eine definierte mittlere Porengröße aufweist. Die mittlere Porengröße des Trägers bezieht sich immer auf jene vor dem Immobilisieren des Polymyxins über hydrophobe Interaktion.

Die mittlere Porengröße kann besonders gut eingestellt werden, wenn der poröse Träger aus einem synthetischen Polymer hergestellt ist. Obwohl einem Fachmann auf dem Gebiet bekannt ist, was unter dem Begriff "mittlere Porengröße" zu verstehen ist und wie die Porösität bzw. die mittlere Porengröße gezielt einstellt werden kann, soll dieser Begriff aus Gründen der Klarheit an dieser Stelle dennoch kurz definiert werden. Die mittlere Porengröße bezieht sich auf den mittleren Durchmesser der Poren. Bei einer gaußschen Größenverteilung der Porendurchmesser eines porösen Materials ist der mittlere Porendurchmesser jener, welchem dem Maximum der Verteilungskurve entspricht. Der mittlere Porendurchmesser kann beispielsweise mittels Stickstoffadsorption (wie beschrieben in Weber et al. 2008; Neutral styrene divinylbenzene copolymers for adsorption of toxins in liver failure. Biomacromolecules 9(4):1322-1328) oder mittels Quecksilberintrusion bestimmt werden. Eingestellt wird die Porengröße eines Polymers durch Variation der Konzentration der beteiligten Monomere bzw. Co-Monomere, des Lösungsmittels oder des Modulators. Je kleiner die Poren des Polymers gewählt sind, umso größer wird die innere Oberfläche des Polymers, die für eine Sorption, insbesondere Adsorption, zur Verfügung steht. Je größer die Poren, umso besser ist die Zugänglichkeit der Poren für größere Moleküle. Ein Herstellungsverfahren für ein synthetisches, hydrophobes Polymer definierter Porengröße, wie es für die Erfindung zur Anwendung kommen kann, ist in der oben genannten Publikation von Weber et al. beschrieben.

Es hat sich herausgestellt, dass eine besonders gute Endotoxinsorption durch das Sorptionsmittel erreicht werden kann, wenn der Träger eine mittlere Porengröße von zumindest 15 nm aufweist. Vorzugsweise weist der Träger eine mittlere Porengröße von zumindest 30 nm auf. Für die klinische Anwendung einer extrakorporalen Blutreinigung ist es jedoch günstig, wenn die mittlere Porengröße des unbeschichteten Trägers nicht größer als 120 nm ist. Die innere Oberfläche des Sorptionsmittels würde ansonsten zu klein werden; die Folge wäre eine Verminderung der Endotoxinsorptionskapazität (Endotoxinadsorptionskapazität). Im Laborversuch (siehe Beispiele weiter unten) wurden sehr gute Ergebnisse erhalten, wenn der unbeschichtete Träger eine mittlere Porengröße von 30-40 nm aufweist.

Erfindungsgemäß weist der unbeschichtete Träger eine mittlere Porengröße von 80-100 nm auf. Bei dieser Porengröße erfolgt die Eliminierung von Endotoxinen aus einer biologischen Flüssigkeit mit besonders hoher Geschwindigkeit und hoher Effizienz. Es ist daher nur eine geringe Menge an Sorptionsmittel notwendig, um eine große Menge Endotoxin zu binden. Beispielsweise kann die Konzentration des erfindungsgemäßen Sorptionsmittels, wenn als Suspension in einem extrakorporalen Plasmakreislauf angewendet, 1%ig (Gew.-% Vol%) gewählt werden. Ein extrakorporaler Plasmakreislauf, in welchem eine Suspension eines Mikropartikel-förmigen Sorptionsmittels enthalten ist, stellt einen zentralen Bestandteil eines Microspheres-based Detoxification Systems (MDS) dar. Ein MDS ist aus der EP 0776223 B und der US 5855782 bekannt geworden.

Darüber hinaus ist auch die Form des erfindungsgemäßen Sorptionsmittels beim Sorptionsvorgang von Bedeutung. Bei einer vorteilhaften Variante ist das erfindungsgemäße Sorptionsmittel mikropartikelförmig, wobei die Mikropartikel eine Partikelgröße von 20 µm oder kleiner aufweisen.

Die Partikelgröße beeinflusst die Kinetik der Adsorption. Darüber hinaus liegt bei einer kleinen Partikelgröße ein großes Oberflächen/VolumenVerhältnis vor.

Die Mikropartikel finden insbesondere in einem MDS Anwendung, welches zuvor bereits erwähnt wurde. Die Mikropartikel zirkulieren als Suspension in einem Reinigungskreislauf (Plasmakreislauf) auf der Filtratseite eines Membranfilters. Sollte der Membranfilter allerdings undicht werden, besteht die Gefahr, dass Mikropartikel in den extrakorporalen Blutkreislauf und weiter in den Körper des Patienten gelangen und dort zu einer Lungenembolie führen. Aus diesem Grunde ist es bei einer weiteren Untervariante von Vorteil, wenn die Mikropartikel eine Partikelgröße von 8 µm oder kleiner, idealerweise 5 µm oder kleiner, aufweisen, da bei diesen kleinen Partikelgrößen die Gefahr einer Lungenembolie umgangen werden kann.

Das erfindungsgemäße Sorptionsmittel ist hauptsächlich für die Anwendung in der extrakorporalen Blutreinigung (Apherese) vorgesehen.

In einer wichtigen Anwendungsform der Erfindung kann das Sorptionsmittel als Füllmaterial für eine Sorptionseinrichtung Anwendung finden. Die Sorptionseinrichtung kann als Säule oder Kartusche ausgebildet sein. Je nachdem, welche Blutreinigungsvorrichtung bzw. welches Blutreinigungsverfahren (Hämoperfusion, Plasmapherese/Plasmasorption) angewendet wird, kann die Sorptionseinrichtung blutseitig in einem extrakorporalen Blutkreislauf oder in einem Plasmakreislauf auf der Filtratseite angeordnet sein. Die biologische Flüssigkeit (Blut oder Blutplasma) passiert die Sorptionseinrichtung, wobei die Endotoxine an die immobilisierten Polymyxin-Moleküle des Sorptionsmittels binden. Das gereinigte Blut bzw. Plasma wird dem Patienten wieder zugeführt.

Eine weitere Einsatzmöglichkeit betrifft einen Plasmakreislauf, in welchem das Sorptionsmittel als Suspension im Plasma verteilt vorliegt. Ein Beispiel für einen solchen Plasmakreislauf findet sich als Vorrichtungselement in einem oben beschriebenen MDS. Das in einem Plasmakreislauf in Suspension vorliegende Sorptionsmittel ist vorzugsweise mikropartikelförmig.

Obwohl das erfindungsgemäße Endotoxin-Sorptionsmittel in erster Linie für die Anwendung in der extrakorporalen Blutreinigung (Apherese) vorgesehen ist, ist ein Einsatz in der Chromatographie ebenfalls denkbar. So kann das Sorptionsmittel als Füllmaterial für Chromatographiesäulen zum Reinigen von Endotoxin-belastetem Blut oder Blutplasma eingesetzt werden. Auch andere Anwendungen zum Entfernen von Endotoxinen aus biologischen Flüssigkeiten oder Wasser sind denkbar.

Das erfindungsgemäße Sorptionsmittel ist besonders zur Behandlung einer Sepsis geeignet.

Ferner wird hierin ein Verfahren zum Entfernen von Endotoxinen aus einer biologischen Flüssigkeit beschrieben, welches nicht Gegenstand der Erfindung ist. Bei dem Verfahren wird eine mit Endotoxinen verunreinigte biologische Flüssigkeit mit dem erfindungsgemäßen Sorptionsmittel in Kontakt gebracht. Wie oben beschrieben kann die biologische Flüssigkeit eine Sorptionseinrichtung, welche das Sorptionsmittel enthält, passieren. Das Sorptionsmittel kann aber auch in der biologischen Flüssigkeit suspendiert sein. Ein Beispiel für letzteres ist das zuvor beschriebene MDS. Die biologische Flüssigkeit kann Blut oder Blutplasma sein.

Die vorliegende Erfindung wird im Folgenden anhand von nicht einschränkenden Beispielen näher erläutert.

### 1. BEISPIEL 1: Herstellung erfindungsgemäßer Sorptionsmittel (Adsorber).

Zum Herstellen erfindungsgemäßer Sorptionsmittel wurden neutrale, hydrophobe Polystyrol-Divinylbenzol-Copolymere unterschiedlicher Porengröße mit Polymyxin B beschichtet, d.h. Polymyxin B wurde über hydrophobe Wechselwirkungen an den äußeren und inneren Oberflächen der Polystyrol-Divinylbenzol-Copolymere adsorbiert.

### 1.1. Bereitstellen neutraler, hydrophober Polymere:

Polystyrol-Divinylbenzol-Copolymere (kurz als "Polymer" oder "Träger" oder "unbeschichteter Adsorber" bezeichnet) unterschiedlicher mittlerer Porengröße sind in der Tabelle 1 aufgelistet.

**Tabelle 1: Polystyrol-Divinylbenzol-Copolymere**

| Bezeichnung | Mittlere Porengröße [nm] |
|---|---|
| #1822 | 15-20 |
| #1823 | 15-20 |
| #1824 | 30-40 |
| #1825 | 80-100 |
| #1826 | 80-100 |

Die Partikelgröße der Polymere betrug 5 µm +/- 3-4 µm.

### 1.2. Polymyxin-Beschichtung der Polystyrol-Divinylbenzol-Copolymere:

Zum Herstellen der erfindungsgemäßen Adsorber wurden die in Tabelle 1 aufgelisteten Polymere mit Polymyxin B beschichtet.

Polymyxin B (PMB) wurde von der Firma Sigma (Kat.Nr.: 81334, Lot: 1348744) bezogen. Für die Beschichtung wurde eine Polymyxin-B-Lösung (PMB-Lösung) hergestellt, wobei 50 mg PMB in 10 ml LAL-Wasser gelöst wurden.

Je 1g Polymer Feuchtgewicht wurde mit 5 ml PMB-Lösung versetzt und 60 min in einem Enviro Genie® Schüttler bei Raumtemperatur inkubiert.

Danach wurde bei 4000g für 15 min abzentrifugiert, der Überstand abgehoben und das Sediment mit 10 ml 0,9% NaCl versetzt und gevortext. Dieser Schritt wurde 3-5 mal wiederholt. Anschließend wurde noch einmal bei 4000g für 15 min abzentrifugiert, der Überstand abgehoben und eine 50%ige Adsorbersuspension in pyrogrenfreiem 0,9%NaCl hergestellt. Die Polymyxin B-beschichteten Adsorber sind in Tabelle 2 aufgelistet.

**Tabelle 2: Polymyxin B-beschichtete Adsorber**

| Bezeichnung des Polymyxin B-beschichteten Adsorbers | Mittlere Porengröße [nm] des unbeschichteten Adsorbers |
|---|---|
| #1822+PMB | 15-20 |
| #1823+PMB | 15-20 |
| #1824+PMB | 30-40 |
| #1825+PMB | 80-100 |
| #1826+PMB | 80-100 |

### 2. BEISPIEL 2: Endotoxinadsorption - Batchtest

Polymyxin B-beschichtete Adsorber #1825+PMB und #1826+PMB mit einer mittleren Porengröße (PG) der Polymere von 80-100 nm (siehe 1. Beispiel 1 - Tabelle 2) wurden mit entsprechenden unbeschichteten Adsorbern #1825 und #1826 hinsichtlich der Endotoxinbindung verglichen.

### 2.1. Adsorber und Adsorbervorbereitung:

Die folgenden Adsorber (PG = 80-100) wurden gemäß dem Protokoll aus des Beispiels 1 vorbereitet:
1) #1825
2) #1826
3) #1825+PMB (PMB-beschichtet)
4) #1826+PMB (PMB-beschichtet)

Die Polymyxin B-beschichteten und die unbeschichteten Adsorber wurden - wie in 1.2. beschrieben - 5x mit NaCl pf. gewaschen. Zuletzt wurde eine 50%ige Adsorbersuspension in pyrogenfreiem NaCl hergestellt.

### 2.2. Heparinplasma:

25 ml Heparinplasma wurde von einem Spender (5 x 9ml Vollblutabnahme) bezogen.

### 2.3. Endotoxinlösung:

*LPS Pseudomonas aeruginosa* (Fa. Sigma, L7018, Charge 109H4043). Die portionierten (à 100µl 10 ⁻³g/ml (1mg/ml)) und bei -70°C in Mikrozentrifugenröhrchen gelagerten Endotoxine wurden aufgetaut und mit 900µl LAL-Wasser versetzt. Anschließend wurden die Endotoxine in NaCl bis zur erforderlichen Konzentration von *50 ng*/*ml* (= Endotoxinlösung; ET-Lösung)weiter verdünnt.

### 2.4. Testansätze:

Bei dem Ansatz des Tests erfolgte eine weitere Verdünnung der Endotoxinlösung bis zu einer Endkonzentration von 5 ng/ml. Die Adsorberendkonzentration lag bei 10%. Für die Testansätze wurden 50%ige Adsorbersuspension (Volumen Ads.Suspension), Heparinplasma (Plasma) und die Endotoxinlösung [50 ng/ml] (ET-Lösung) gemäß den Angaben der Tabelle 3 in Teströhrchen pipettiert. Als Kontrolle wurden ein Teströhrchen ohne Adsorber (Kontrolle) sowie ein Teströhrchen mit unbehandeltem Heparinplasma (Plasma) mitgeführt.

**Tabelle 3: Testansätze**

| Adsorber | Vol. Ads.Suspension | Plasma | ET-Lösung |
|---|---|---|---|
| #1825 | 600 µL | 2100 µL | 300 µL |
| #1826 | 600 µL | 2100 µL | 300 µL |
| #1825+PMB | 600 µL | 2100 µL | 300 µL |
| #1826+PMB | 600 µL | 2100 µL | 300 µL |
| Kontrolle | 100 µL NaCl | 800 µL | 100 µL |
| Plasma | 0 | 100 µL | 0 |

Die Testansätze wurden im Enviro-Genie - Schüttler bei 37°C für 60min inkubiert.

### 2.5. Probennahme:

Alle Schritte des Batchtests und des Lal-Tests wurden in Glas-Lal-Teströhrchen (T100, mit Plastikkappe) der Fa. Chromogenix durchgeführt (auch Verdünnungen und Standardkurven). Nur die Probennahme zum Abzentrifugieren erfolgte in pf. Mikrozentrifugenröhrchen.

Nach 5, 15 und 60 min Inkubationszeit wurden die Röhrchen gevortext. Je 0,2 ml Probe wurden entnommen und in pf. Mikrozentrifugenröhrchen überführt. Die Proben wurden in einer Zentrifuge (Eppifuge) für 10min bei 11000g abzentrifugiert. 100 µl Überstand wurden in Glas-Lal-Teströhrchen überführt und im Lal-Test getestet.

| **2.6. Materialien:** | | **Charge:** |
|---|---|---|
| Mikrotiterplatten MT 1007 | Fa. CoaChrom | 02430103 |
| Lal-Test Tubes T 200 | Ch. River Endosafe | 53351 D |
| Combitips plus 2,5 Biopur | Eppendorf | V126339 |
| Pipettentips | Eppendorf | V125542M |
| Pipettentips | Eppendorf | W130324Q |
| NaCl 0,9% | Mayerhofer | |
| Mikrozentrifugentubes | Greiner | 05200108 |
| Charles River Endosafe Endochrome | Kit.Lot: | W3322CTK6 |

### 2.7. Ergebnis:

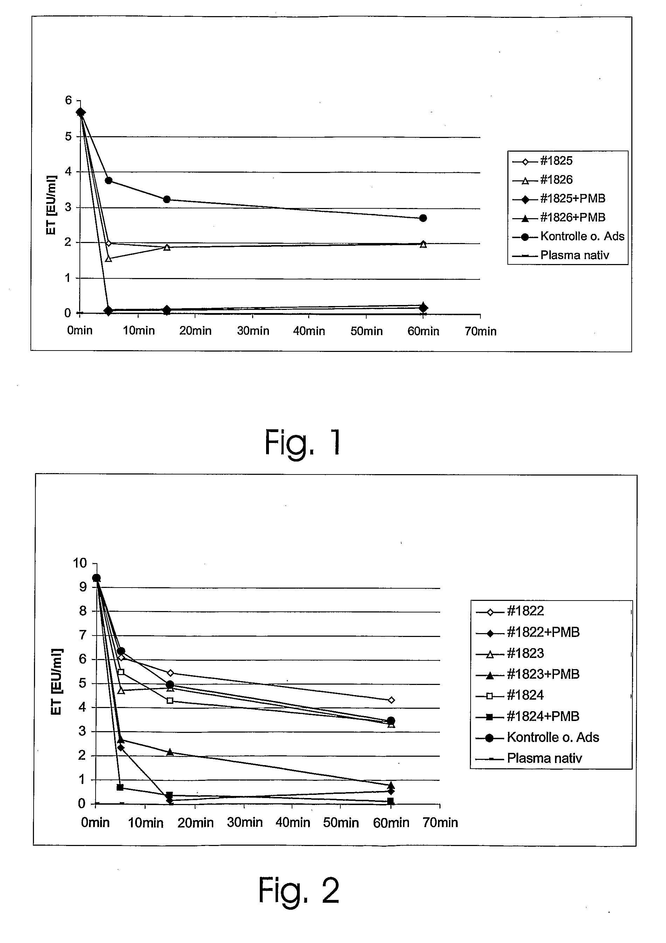

Das Ergebnis ist in der Tabelle 4 aufgelistet und in der Fig. 1 dargestellt, wobei die in Fig. 1 gezeigten Kurven die Endotoxin (ET)-Adsorption (in Endotoxin Units (EUI/ml) PMB-beschichteter Adsorber (mittlere Porengröße 80-100 nm) im Zeitverlauf darstellt.

**Tabelle 4: Ergebnis Batchtest - Endotoxinadsorption**

| | 0min | 5min | 15min | 60min |
|---|---|---|---|---|
| #1825 | 5,662 | 1,98 | 1,88 | 2,00 |
| #1826 | 5,662 | 1,539 | 1,88 | 1,98 |
| #1825+PMB | 5,662 | 0,0665 | 0,09 | 0,17 |
| #1826+PMB | 5,662 | 0,09 | 0,13 | 0,26 |
| Kontrolle o. A | 5,662 | 3,749 | 3,22 | 2,70 |
| Plasma nativ | | | | 0 |

### 3. BEISPIEL 3: Endotoxinadsorption - Batchtest

Polymyxin B-beschichtete Adsorber #1822+PMB, #1823+PMB und #1824+PMB mit einer mittleren Porengröße (PG) von 15-20 nm bzw. 30-40 nm wurden mit entsprechenden unbeschichteten Adsorbern #1822, #1823 und #1824 hinsichtlich der Endotoxinbindung verglichen.

### 3.1. Adsorber und Adsorbervorbereitung:

Die folgenden Adsorber wurden gemäß dem Protokoll des Beispiels 1 vorbereitet:
1) #1822
2) #1823
2) #1824
3) #1822+PMB (PMB-beschichtet)
4) #1823+PMB (PMB-beschichtet)
5) #1824+PMB (PMB-beschichtet)

Die Polymyxin B-beschichteten und die unbeschichteten Adsorber wurden - wie in Abschnitt 1.2. beschrieben - 5x mit NaCl pf. gewaschen. Zuletzt wurde eine 50%ige Adsorbersuspension in pyrogenfreiem NaCl hergestellt.

### 3.2. Heparinplasma und Endotoxinlösung: entsprechend den Abschnitten 2.2. und 2.3.

### 3.3. Testansätze:

Bei dem Ansatz des Tests erfolgte eine weitere Verdünnung der Endotoxinlösung bis zu einer Endkonzentration von 5 ng/ml. Die Adsorberendkonzentration lag bei 10%. Für die Testansätze wurden 50%ige Adsorbersuspension (Vol.Ads.Suspension), Heparinplasma (Plasma) und Endotoxinlösung [50 ng/ml] (ET-Lösung) gemäß den Angaben der Tabelle 5 in Teströhrchen pipettiert. Als Kontrolle wurden ein Teströhrchen ohne Adsorber (Kontrolle o. Ads.) sowie ein Teströhrchen mit unbehandeltem Heparinplasma (Plasma nativ) mitgeführt.

**Tabelle 5: Testansätze**

| Adsorber | Vol. Ads. suspension | Plasma | ET-Lösung |
|---|---|---|---|
| # 1822 | 600 µL | 2100 µL | 300 µL |
| # 1822+PMB | 600 µL | 2100 µL | 300 µL |
| # 1823 | 600 µL | 2100 µL | 300 µL |
| # 1823+PMB | 600 µL | 2100 µL | 300 µL |
| # 1824 | 600 µL | 2100 µL | 300 µL |
| # 1824+PMB | 600 µL | 2100 µL | 300 µL |
| Kontrolle o. Ads. | 100 µL NaCl | 800 µL | 100 µL |
| Plasma nativ | 0 | 100 µL | 0 |

Die Testansätze wurden im Enviro-Genie - Schüttler bei 37°C für 60min inkubiert.

### 3.4. Probennahme und Materialen: entsprechend den Abschnitten 2.5. und 2.6.

### 3.5. Ergebnis:

Das Ergebnis ist in der Tabelle 6 aufgelistet und in der Fig. 2 dargestellt, wobei die in Fig. 2 gezeigten Kurven die Endotoxin (ET)-Adsorption (in Endotoxin Units (EU)/ml) PMB-beschichteter Adsorber (mittlere Porengröße 15-20 bzw. 30-40 nm) im Zeitverlauf darstellen.

**Tabelle 6: Ergebnis Batchtest - Endotoxinadsorption**

| EU/ml | 0 min | 5 min | 15 min | 60 min |
|---|---|---|---|---|
| # 1822 | 9,37 | 6,06 | 5,46 | 4,34 |
| # 1822+PMB | 9,37 | 2,34 | 0,18 | 0,53 |
| # 1823 | 9,37 | 4,71 | 4,84 | 3,33 |
| # 1823+PMB | 9,37 | 2,65 | 2,16 | 0,77 |
| # 1824 | 9,37 | 5,47 | 4,30 | 3,41 |
| # 1824+PMB | 9,37 | 0,68 | 0,36 | 0,14 |
| Kontrolle o. Ads. | 9,37 | 6.33 | 4,95 | 3,45 |
| Plasma nativ | 0 | 0 | 0 | 0 |

### 4. BEISPIEL 4: Endotoxinadsorption - Batchtest bei verschiedenen Adsorberendkonzentrationen in Heparinplasma.

Polymyxin B-beschichtete Adsorber #1823+PMB (mittlere Porengröße 15-20 nm) und #1826+PMB (mittlere Porengröße 80-100 nm) wurden mit entsprechenden unbeschichteten, unbehandelten Adsorbern #1823 und #1826 hinsichtlich der Endotoxinbindung bei verschiedenen Adsorberendkonzentrationen, nämlich 10%, 4%, 2% und 1%, verglichen.

### 4.1. Adsorber und Adsorbervorbereitung:

Die folgenden Adsorber wurden gemäß dem Protokoll des Beispiels 1 vorbereitet:
1) #1823
2) #1826
3) #1823+PMB (PMB-beschichtet)
4) #1826+PMB (PMB-beschichtet)

Die Polymyxin B-beschichteten und die unbeschichteten Adsorber wurden - wie in 1.2. beschrieben - 5x mit NaCl pf. gewaschen. Schließlich wurde eine 50%ige Adsorbersuspension in pyrogenfreiem NaCl hergestellt.

### 4.2. Heparinplasma und Endotoxinlösung: entsprechend den Abschnitten 2.2. und 2.3.

### 4.3. Testansätze:

Bei dem Ansatz des Tests erfolgt eine weitere Verdünnung der Endotoxinlösung bis zu einer Endkonzentration von 5 ng/ml.

Insgesamt wurden vier Testansätze A, B, C und D unterschiedlicher Adsorberendkonzentration hergestellt, wobei für jeden Polymyxin B-beschichteten Adsorber ein Doppelansatz hergestellt wurde. Für die Testansätze wurden 50%ige Adsorbersuspension (Vol. Ads. suspension), Heparinplasma (Plasma) und Endotoxinlösung (ET-Lösung) gemäß den Angaben der Tabellen 7 - 10 in Teströhrchen pipettiert. Als Kontrollen wurden ein Teströhrchen ohne Adsorber (Kontrolle o. Adsorber) sowie ein Teströhrchen mit unbehandeltem Heparinplasma (Plasma) mitgeführt.

**Tabelle 7: Testansatz A - Endotoxinkonzentration 5 ng/ml, Adsorberendkonzentration 10%**

| Adsorber | Vol. Ads. suspension | Plasma | ET-Lösung |
|---|---|---|---|
| #1823 | 660 µL | 2367 µL | 300 µL |
| #1823+PMB | 660 µL | 2367 µL | 300 µL |
| # 1826 | 660 µL | 2367 µL | 300 µL |
| # 1826+PMB | 660 µL | 2367 µL | 300 µL |
| Kontrolle o. Ads. | 0 | 900 µL | 100 µL |
| Plasma | 0 | 100 µL | 0 |

**Tabelle 8: Testansatz B - Endotoxinkonzentration 5 ng/ml, Adsorberendkonzentration 4%**

| Adsorber | Vol. Ads. suspension | Plasma | ET-Lösung |
|---|---|---|---|
| #1823 | 250 µL | 2575 µL | 300 µL |
| #1823+PMB | 250 µL | 2575 µL | 300 µL |
| # 1826 | 250 µL | 2575 µL | 300 µL |
| # 1826+PMB | 250 µL | 2575 µL | 300 µL |
| Kontrolle o. Ads. | 0 | 900 µL | 100 µL |
| Plasma | 0 | 100 µL | 0 |

**Tabelle 9: Testansatz C - Endotoxinkonzentration 5 ng/ml, Adsorberendkonzentration 2 %**

| Adsorber | Vol. Ads. suspension | Plasma | ET-Lösung |
|---|---|---|---|
| #1823 | 122 µL | 2639 µL | 300 µL |
| #1823+PMB | 122 µL | 2639 µL | 300 µL |
| # 1826 | 122 µL | 2639 µL | 300 µL |
| # 1826+PMB | 122 µL | 2639 µL | 300 µL |
| Kontrolle o. Ads. | 0 | 900 µL | 100 µL |
| Plasma | 0 | 100 µL | 0 |

**Tabelle 10: Testansatz D - Endotoxinkonzentration 5 ng/ml, Adsorberendkonzentration 1 %**

| Adsorber | Vol. Ads. suspension | Plasma | ET-Lösung |
|---|---|---|---|
| #1823 | 60 µL | 2670 µL | 300 µL |
| #1823+PMB | 60 µL | 2670 µL | 300 µL |
| #1826 | 60 µL | 2670 µL | 300 µL |
| # 1826+PMB | 60 µL | 2670 µL | 300 µL |
| Kontrolle o. Ads. | 0 | 900 µL | 100 µL |
| Plasma | 0 | 100 µL | 0 |

Die Testansätze wurden im Enviro-Genie - Schüttler bei 37°C für 60min inkubiert.

### 4.4. Probennahme:

Alle Schritte des Batchtests und des Lal-Tests wurden in Glas-Lal-Teströhrchen (T100, mit Plastikkappe) der Fa. Chromogenix durchgeführt (auch Verdünnungen und Standardkurven). Nur die Probennahme zum Abzentrifugieren erfolgte in pf. Mikrozentrifugenröhrchen. Nach 5, 15 und 60 min Inkubationszeit wurden die Röhrchen gevortext. A' 0,3ml Probe wurden entnommen und in pf. Mikrozentrifugentubes überführt. Die Proben wurden in einer Zentrifuge (Eppifuge) für 3 min bei 11000g abzentrifugiert. 50 µl Überstand wurden abgehoben, in Glas-Lal-Teströhrchen zu 450 µl LAL-Wasser überführt, sofort bei 75°C für 5 min inkubiert, auf eine Mikrotiterplatte aufgetragen und im Lal-Test getestet.

### 4.5. Materialen: entsprechend den Abschnitten 2.5. und 2.6.

### 4.6. Ergebnis:

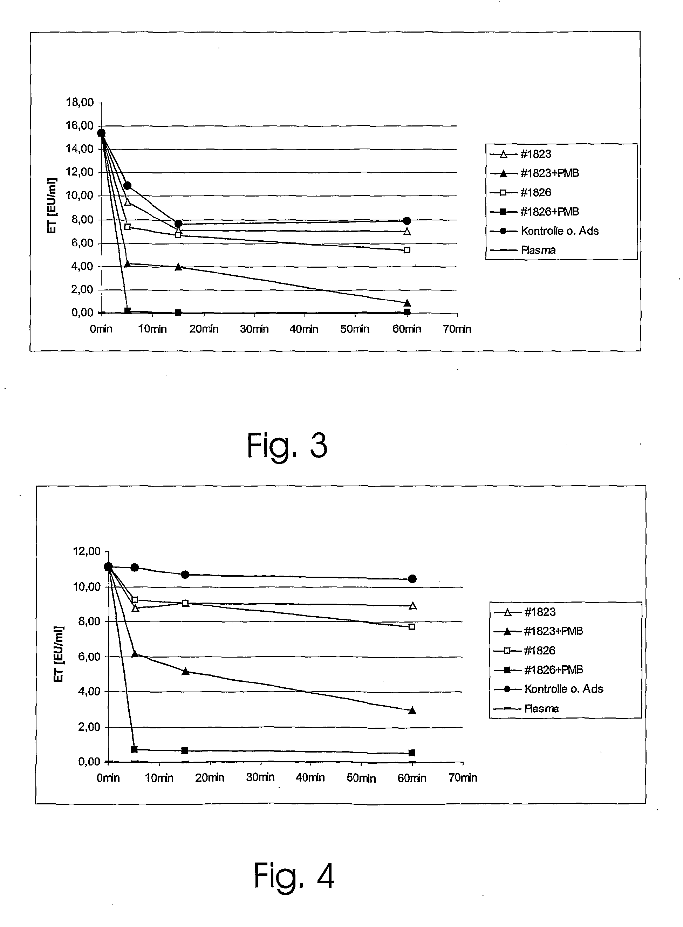

Die Ergebnisse sind in den Tabellen 11, 12, 13 und 14 aufgelistet und in **Fig. 3**, **Fig. 4**, **Fig. 5** und **Fig. 6** dargestellt. Die Endotoxin (ET)-Adsorption (in Endotoxin Units (EU)/ml) ist im Zeitverlauf dargestellt. Die Tabellen und Figuren zeigen:

Tabelle 11 sowie **Fig. 3****:** Ergebnisse des Batchtests (Testansatz A) bei einer 10%igen Adsorberkonzentration:

**Tabelle 11:**

| EU/ml | 0 min | 5 min | 15 min | 60 min |
|---|---|---|---|---|
| #1823 | 15,39 | 9,55 | 7,07 | 7,03 |
| #1823+PMB | 15,39 | 4,28 | 4,00 | 0,90 |
| #1826 | 15,39 | 7,33 | 6,70 | 5,37 |
| #1826+PMB | 15,39 | 0,19 | 0 | 0 |
| Kontrolle o. Ads. | 15,39 | 10,91 | 7,57 | 7,91 |
| Plasma nativ | 0 | x | x | 0 |

Tabelle 12 sowie **Fig. 4****:** Ergebnisse des Batchtests (Testansatz B) bei einer 4%igen Adsorberkonzentration:

**Tabelle 12:**

| EU/ml | 0 min | 5 min | 15 min | 60 min |
|---|---|---|---|---|
| #1823 | 11,12 | 8,74 | 9,03 | 8,94 |
| #1823+PMB | 11,12 | 6,16 | 5,14 | 2,96 |
| #1826 | 11,12 | 9,21 | 9,02 | 7,70 |
| #1826+PMB | 11,12 | 0,69 | 0,62 | 0,35 |
| Kontrolle o. Ads. | 11,12 | 11,10 | 10,69 | 10,45 |
| Plasma nativ | 0 | x | x | 0 |

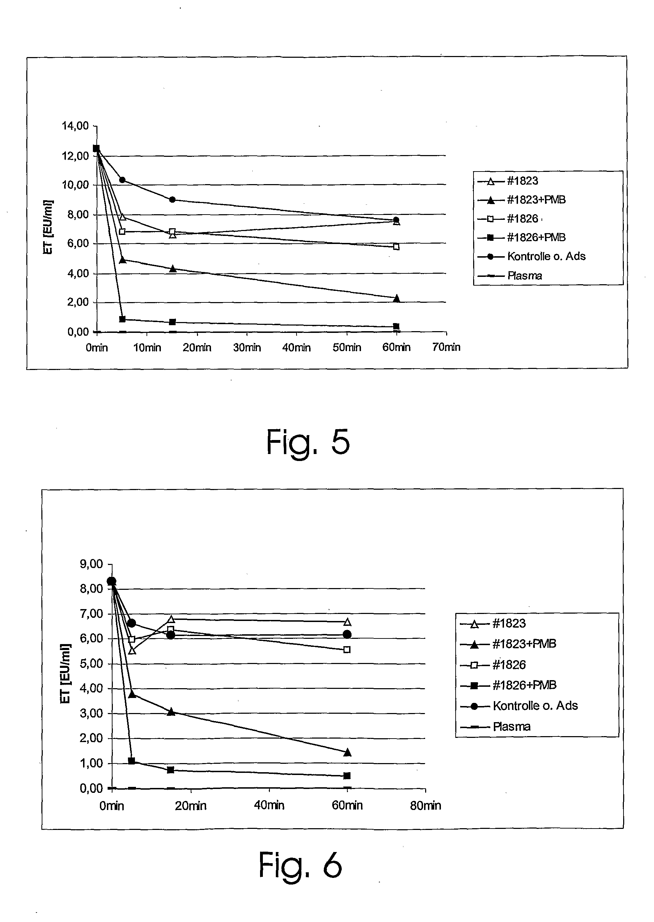

Tabelle 13 sowie **Fig. 5****:** Ergebnisse des Batchtests (Testansatz C) bei einer 2%igen Adsorberkonzentration:

**Tabelle 13:**

| EU/ml | 0 min | 5 min | 15 min | 60 min |
|---|---|---|---|---|
| #1823 | 12,49 | 7,85 | 6,66 | 7,48 |
| #1823+PMB | 12,49 | 4,93 | 4,33 | 2,33 |
| #1826 | 12,49 | 6,83 | 6,82 | 5,77 |
| #1826+PMB | 12,49 | 0,91 | 0,65 | 0,39 |
| Kontrolle o. Ads. | 12,49 | 10,35 | 9,00 | 7,59 |
| Plasma nativ | 0 | x | x | 0 |

Tabelle 14 sowie **Fig. 6****:** Ergebnisse des Batchtests (Testansatz D) bei einer 1%igen Adsorberkonzentration:

**Tabelle 14:**

| EU/mL | 0 min | 5 min | 15 min | 60 min |
|---|---|---|---|---|
| # 1823 | 8,29 | 5,54 | 6,80 | 6,65 |
| #1823+PMB | 8,29 | 3,78 | 3,09 | 1,43 |
| # 1826 | 8,29 | 5,95 | 6,33 | 5,54 |
| # 1826+PMB | 8,29 | 1,07 | 0,75 | 0,47 |
| Kontrolle o. Ads. | 8,29 | 6,61 | 6,11 | 6,11 |
| Plasma nativ | 0 | x | x | 0 |

### 5. BEISPIEL 5: Endotoxinadsorption-Batchtest bei einer Endotoxinkonzentration von 1ng/ml.

Polymyxin B-beschichtete Adsorber (siehe Beispiel 1) #1823+PMB (mittlere Porengröße 15-20 nm), #1824+PMB (mittlere Porengröße 30-40 nm) und #1826+PMB (mittlere Porengröße 80-100 nm) wurden mit entsprechenden unbeschichteten Adsorbern #1823, #1824 und #1826 hinsichtlich der Endotoxinbindung verglichen, wobei die Endotoxinkonzentration im Batchtest 1ng/ml war.

### 5.1. Adsorber und Adsorbervorbereitung:

Die folgenden Adsorber wurden gemäß dem Protokoll des Beispiels 1 vorbereitet:
1) #1823
2) #1824
3) #1826
4) #1823+PMB (PMB-beschichtet)
5) #1824+PMB (PMB-beschichtet)
6) #1826+PMB (PMB-beschichtet)

Die Polymyxin B-beschichteten und die unbeschichteten Adsorber wurden - wie in 1.2. beschrieben - 5x mit NaCl pf. gewaschen. Zuletzt wurde eine 50%ige Adsorbersuspension in pyrogenfreiem NaCl hergestellt.

### 5.2. Heparinplasma entsprechend Abschnitt 2.2.

### 5.3. Endotoxinlösung:

*LPS Pseudomonas aeruginosa* (Fa. Sigma, L7018, Charge 109H4043). Die portionierten (à 100µl 10⁻³g/ml (1mg/ml)) und bei -70°C in Mikrozentrifugenröhrchen gelagerten Endotoxine wurden aufgetaut und mit 900µl LAL-Wasser versetzt. Anschließend wurden die Endotoxine in NaCl bis zur erforderlichen Konzentration von *10 ng*/*ml* (= Endotoxinlösung; ET-Lösung)weiter verdünnt.

### 5.4. Testansätze:

Bei dem Ansatz des Tests erfolgt eine weitere Verdünnung der Endotoxinlösung bis zu einer Endkonzentration von 1 ng/ml. Die Adsorberendkonzentration in den Testansätzen lag bei 1%. Für die Testansätze wurden 50%ige Adsorbersuspension (Ads. Susp.50%), Heparinplasma (Plasma) und Endotoxinlösung [10 ng/ml] (ET-Lösung) gemäß den Angaben der Tabelle 15 in Teströhrchen pipettiert. Als Kontrollen wurden ein Teströhrchen ohne Adsorber (Kontrolle o. Adsorber) sowie ein Teströhrchen mit unbehandeltem Heparinplasma (Plasma nativ) mitgeführt.

**Tabelle 15: Testansätze**

| | Ads.Susp. 50% | Plasma | ET-Lösung |
|---|---|---|---|
| #1823 | 60µl | 2670µl | 300µl |
| #1824 | 60µl | 2670µl | 300µl |
| #1826 | 60µl | 2670µl | 300µl |
| #1823+PMB | 60µl | 2670µl | 300µl |
| #1824+PMB | 60µl | 2670µl | 300µl |
| #1826+PMB | 60µl | 2670µl | 300µl |
| Kontrolle o. Ads | | 900µl | 100µl |
| Plasma nativ | 0 | 100µl | 0 |

Die Testansätze wurden im Enviro-Genie - Schüttler bei 37°C für 60 min inkubiert.

### 5.5. Probennahme und Materialen: entsprechend den Abschnitten 2.5. und 2.6.

### 5.6. Ergebnis:

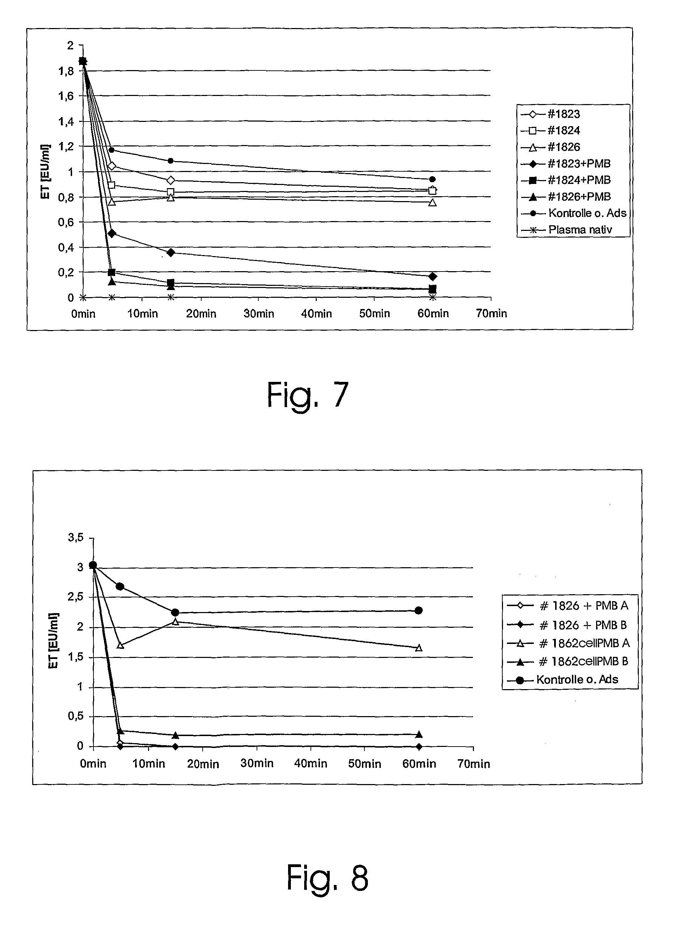

Das Ergebnis ist in der Tabelle 16 aufgelistet und in der **Fig. 7** dargestellt, wobei die **Fig. 7** ein Diagramm der Endotoxin (ET)-Adsorption (Endotoxin Units (EU)/ml) PMB-beschichteter Adsorber bei einer Adsorberkonzentration von 1% und einer Endotoxinkonzentration von 1 ng/ml im Zeitverlauf zeigt.

**Tabelle 16:Ergebnis**

| | 0min | 5min | 15min | 60min |
|---|---|---|---|---|
| #1823 | 1,874 | 1,043 | 0,93 | 0,85 |
| #1824 | 1,874 | 0,89 | 0,83 | 0,84 |
| #1826 | 1,874 | 0,758 | 0,79 | 0,75 |
| #1823+PMB | 1,874 | 0,509 | 0,35 | 0,16 |
| #1824+PMB | 1,874 | 0,195 | 0,115 | 0,067 |
| #1826+PMB | 1,874 | 0,128 | 0,089 | 0,062 |
| Kontrolle o. Ads | 1,874 | 1,169 | 1,08 | 0,93 |
| Plasma nativ | x | x | x | 0 |

### 6. BEISPIEL 6: Austesten der Autoklavierbarkeit Polymyxin B-beschichteter Adsorber.

Die Autoklavierbarkeit eines Polymyxin B-beschichteten Adsorbers #1826+PMB (mittlere Porengröße 80-100 nm) wurde mit einem Adsorber auf Zellulosebasis, bei welchem Polymyxin B kovalent an der Zellulose gebunden ist, verglichen. Der Einfluss des Autoklavierens auf die Endotoxinbindung wurde untersucht.

### 6.1. Adsorber und Adsorbervorbereitung

Zwei 50%ige Adsorbersuspensionen (A und B) eines Polymyxin B-beschichteten Adsorbers #1826+PMB wurden gemäß dem Protokoll des Beispiels 1 hergestellt.

Darüber hinaus wurden zwei 50%ige Adsorbersuspensionen (A und B) eines mit Polymyxin B modifizierten, partikelförmigen Zelluloseadsorbers #1862 Cell+PMB hergestellt (Zellulosepartikel Fischer, Lot: EA 13/1 PCKT 038, mit Epichlorhydrin aktiviert und mit Polymyxin B umgesetzt => kovalente Bindung der Polymyxin B-Moleküle an die Zellulosepartikel).

Die jeweiligen Adsorbersuspensionen A, #1826+PMB A und #1862 Cell+PMB A wurden bei 121°C für 20 min autoklaviert. Die jeweiligen Adsorbersuspensionen B, #1826+PMB B und #1862 Cell+PMB B, wurden nicht autoklaviert:
1) #1826+PMB A (autoklaviert)
2) #1826+PMB B (nicht autoklaviert)
3) #1862 Cell+PMB A (autoklaviert)
4) #1862 Cell+PMB B (nicht autoklaviert)

Von den 50%igen Adsorbersuspensionen wurden jeweils 600µl zu dem vorgelegten Heparinplasma in Lal-Test Tubes (Ch.River Endosafe, pf) T100 verbracht.

### 6.2. Heparinplasma: entsprechend Abschnitt 2.2.

### 6.3. Endotoxinlösung:

*LPS Pseudomonas aeruginosa* (Fa. Sigma, L7018, Charge 109H4043). Die portionierten (je 100µl 10⁻³g/ml (1mg/ml)) und bei -70°C in Mikrozentrifugenröhrchen gelagerten Endotoxine wurden aufgetaut und mit 900µl LAL-Wasser versetzt. Anschließend wurden die Endotoxine in NaCl bis zur erforderlichen Konzentration von *10 ng*/*ml* (= Endotoxinlösung; ET-Lösung)weiter verdünnt.

### 6.4. Testansätze:

Bei dem Ansatz des Tests erfolgt eine weitere Verdünnung der Endotoxinlösung bis zu einer Endkonzentration von 1 ng/ml. Die Adsorberendkonzentration in den Testansätzen lag bei 1%. Für die Testansätze wurden 50%ige Adsorbersuspension (Vol. Ads. suspension), Heparinplasma (Plasma) und Endotoxinlösung [10 ng/ml] (ET-Lösung) gemäß den Angaben der Tabelle 17 in Teströhrchen pipettiert. Als Kontrollen wurden ein Teströhrchen ohne Adsorber (Kontrolle o. Ads.) sowie ein Teströhrchen mit unbehandeltem Heparinplasma (Plasma nativ) mitgeführt.

**Tabelle 17: Testansätze**

| Adsorber | Vol. Ads. suspension | Plasma | ET-Lösung |
|---|---|---|---|
| #1826 + PMB A | 60 µL | 2670 µL | 300 µL |
| #1826 + PMB B | 60 µL | 2670 µL | 300 µL |
| # 1862 Cell + PMB A | 60 µL | 2670 µL | 300 µL |
| # 1862 Cell + PMB B | 60 µL | 2670 µL | 300 µL |
| Kontrolle o. Ads. | 0 | 900 µL | 100 µL |
| Plasma nativ | 0 | 100 µL | 0 |

Die Testansätze wurden im Enviro-Genie-Schüttler bei 37°C für 60 min inkubiert.

### 6.5. Probennahme:

Alle Schritte des Batchtests und des Lal-Tests wurden in Glas-Lal-Teströhrchen (T100, mit Plastikkappe) der Fa. Chromogenix durchgeführt (auch Verdünnungen und Standardkurven). Nur die Probennahme zum Abzentrifugieren erfolgte in pf. Mikrozentrifugenröhrchen.

Nach 5, 15 und 60 min Inkubationszeit wurden die Röhrchen gevortext. Jeweils 0,2 ml der Probe wurden entnommen und in pf. Mikrozentrifugenröhrchen überführt. Die Proben wurden in einer Tischzentrifuge (Eppifuge) für 10min bei 11000g abzentrifugiert. 100 µl Überstand wurden in Glas-Lal-Teströhrchen überführt und im Lal-Test getestet (Lal-Test: Charles River Endosafe Endochrome, Kit.Lot: Y 1892 EK1)

### 6.6. Ergebnis:

Das Ergebnis ist in der Tabelle 18 aufgelistet und in der Fig. 8 dargestellt, wobei die in Fig. 8 gezeigten Kurven die Endotoxin (ET)-Adsorption (in Endotoxin Units (EU)/ml) der autoklavierten und der nicht-autoklavierten Adsorber bzw. der Zelluloseadsorber sowie der Kontrollen im Zeitverlauf darstellen.

**Tabelle 18: Ergebnis**

| EU/ml | 0 min | 5 min | 15 min | 60 min |
|---|---|---|---|---|
| #1826 + PMB A | 3,047 | 0,062 | 0,00 | 0,00 |
| #1826 + PMB B | 3,047 | 0,00 | 0,00 | 0,00 |
| # 1862 Cell + PMB A | 3,047 | 1,696 | 2,10 | 1,65 |
| # 1862 Cell + PMB B | 3,047 | 0,273 | 0,20 | 0,21 |
| Kontrolle o. Ads. | 3,047 | 2,684 | 2,24 | 2,27 |
| Plasma nativ | x | x | x | 0 |

Die erfindungsgemäßen Adsorber #1826+PMB A bzw. #1826+PMB B wiesen sowohl vor als auch nach dem Autoklavieren eine gute Endotoxin-Adsorption auf.

Das Autoklavieren des Zelluloseadsorbers hingegen bewirkte eine signifikante Verschlechterung der Endotoxin-Adsorption.

## Patentansprüche

1. Sorptionsmittel zum Entfernen von Endotoxinen aus einer biologischen Flüssigkeit, wobei das Sorptionsmittel einen wasserunlöslichen, porösen Träger und Polymyxin, welches am Träger immobilisiert ist, aufweist, wobei der Träger eine nicht-ionische, hydrophobe Oberfläche aufweist und Polymyxin über hydrophobe Interaktion an der Oberfläche des Trägers immobilisiert ist,
**dadurch gekennzeichnet, dass**
dass der Träger eine mittlere Porengröße von 80 - 100 nm aufweist, wobei sich die mittlere Porengröße auf den mittleren Durchmesser der Poren bezieht.

2. Sorptionsmittel nach Anspruch 1, **dadurch gekennzeichnet, dass** der Träger ein hydrophobes Polymer ist.

3. Sorptionsmittel nach Anspruch 2, **dadurch gekennzeichnet, dass** das hydrophobe Polymer ein vernetztes Polystyrol-Polymer, insbesondere ein Polystyrol-Divinylbenzol-Copolymer ist.

4. Sorptionsmittel nach einem der Ansprüche 1 bis 3, **dadurch gekennzeichnet, dass** das Sorptionsmittel die Form von Mikropartikeln hat, wobei die Mikropartikel eine Partikelgröße von 20µm oder kleiner, vorzugsweise eine Partikelgröße von 8 µm oder kleiner, idealerweise 5 µm oder kleiner, aufweisen.

5. Sorptionseinrichtung, enthaltend ein Sorptionsmittel nach einem der Ansprüche 1 bis 4.

6. Blutplasmakreislauf, enthaltend eine Suspension eines Sorptionsmittels nach einem der Ansprüche 1 bis 4.

7. Sorptionsmittel nach einem der Ansprüche 1 bis 4 zur Anwendung in der Behandlung einer Sepsis.

8. Microspheres-based Detoxification System (MDS) umfassend ein Sorptionsmittel nach einem der Ansprüche 1 bis 4.

## Claims

1. A sorption agent for removing endotoxins from a biological fluid, the sorption agent having a water-insoluble, porous carrier and polymyxin, which is immobilized on the carrier, wherein the carrier has a non-ionic, hydrophobic surface and polymyxin is immobilized on the surface of the carrier via hydrophobic interaction,
**characterized in that**
the carrier has a mean pore size of approximately 80 - 100 nm, wherein the mean pore size relates to the mean diameter of the pores.

2. The sorption agent according to Claim 1, **characterized in that** the carrier is a hydrophobic polymer.

3. The sorption agent according to Claim 2, **characterized in that** the hydrophobic polymer is a cross-linked polystyrene polymer, in particular a polystyrene-divinyl benzene copolymer.

4. The sorption agent according to one of Claims 1 to 3, **characterized in that** the sorption agent has the form of microparticles, wherein the microparticles have a particle size of 20 µm or less, preferably a particle size of 8 µm or less, ideally 5 µm or less.

5. A sorption apparatus, containing a sorption agent according to one of Claims 1 to 4.

6. A plasma circuit, comprising a suspension of a sorption agent according to one of Claims 1 to 4.

7. The sorption agent according to one of Claims 1 to 4 for use in the treatment of a sepsis.

8. Microspheres-based Detoxification System (MDS) comprising a sorption agent according to one of Claims 1 to 4.

## Revendications

1. Agent de sorption pour l'élimination des endotoxines d'un fluide biologique, l'agent de sorption présentant un support poreux non hydrosoluble et de la polymyxine, qui est immobilisée sur le support, le support présentant une surface non ionique hydrophobe et la polymyxine étant immobilisée par interaction hydrophobe sur la surface du support,
**caractérisé en ce que**
le support présente une taille moyenne de pore de 80 à 100 nm, la taille moyenne de pore se rapportant au diamètre moyen des pores.

2. Agent de sorption selon la revendication 1, **caractérisé en ce que** le support est un polymère hydrophobe.

3. Agent de sorption selon la revendication 2, **caractérisé en ce que** le polymère hydrophobe est un polymère de polystyrène réticulé, en particulier un copolymère de polystyrène - divinylbenzène.

4. Agent de sorption selon une des revendications 1 à 3, **caractérisé en ce que** l'agent de sorption prend la forme de microparticules, lesdites microparticules présentant une taille de particule de 20 µm ou moins, de préférence une taille de particules de 8 µm ou moins, idéalement de 5 µm ou moins.

5. Dispositif de sorption contenant un agent de sorption selon l'une des revendications 1 à 4.

6. Circulation de plasma sanguin, contenant une suspension d'un agent de sorption d'après une des revendications 1 à 4.

7. Agent de sorption selon une des revendications 1 à 4 pour l'utilisation dans le traitement d'une septicémie.

8. Système de détoxification à base de microsphères (MDS) comprenant un agent de sorption selon une des revendications 1 à 4.
